# FASCICULE DE BREVET EUROPEEN

(11) **EP 2 986 347 B1**
(45) Date de publication et mention de la délivrance du brevet: **31.07.2019**
(21) Numéro de dépôt: 14722277.2
(22) Date de dépôt: 15.04.2014
(51) Int. Cl.: A61Q 19/00, A61K 8/99

(54) **APPLICATIONS COSMETIQUES DE LACTOBACILLUS PENTOSUS**
KOSMETISCHE VERWENDUNGEN VON LACTOBACILLUS PENTOSUS
COSMETIC APPLICATIONS OF LACTOBACILLUS PENTOSUS

(30) Priorité: 15.04.2013 FR 1353395
(43) Date de publication de la demande: 24.02.2016
(73) Titulaire: Greentech, 63360 St Beauzire (FR)
(72) Inventeur: RIOS, Laurent, F-63570 Auzat La Combelle (FR); TROPEL, David, F-63270 Vic Le Comte (FR); BERTHON, Jean-Yves, Antonin, F-63540 Romagnat (FR); CHAISEMARTIN, Laurent, F-63270 Vic Le Comte (FR)
(74) Mandataire: Agasse, Stéphane
(86) Numéro de dépôt international: PCT/FR2014/050914
(87) Numéro de publication internationale: WO 2014/170595

(56) Documents cités:
- EP-A1- 2 412 797
- WO-A1-2011/110918
- DATABASE WPI Week 200830 Thomson Scientific, London, GB; AN 2008-E38092 XP002717722, & JP 2008 013502 A (SUNSTAR CHEM IND CO LTD) 24 janvier 2008 (2008-01-24)
- DATABASE WPI Week 200739 Thomson Scientific, London, GB; AN 2007-408919 XP002717723, & JP 2007 126399 A (SUNTORY LTD) 24 mai 2007 (2007-05-24)
- DATABASE WPI Week 200414 Thomson Scientific, London, GB; AN 2004-140523 XP002717724, & KR 2003 0064462 A (BIOTOPIA CO LTD) 2 août 2003 (2003-08-02)
- NONAKA YUJI ET AL: "Antiallergic effects of Lactobacillus pentosus strain S-PT84 mediated by modulation of Th1/Th2 immunobalance and induction of IL-10 production", INTERNATIONAL ARCHIVES OF ALLERGY AND IMMUNOLOGY,, vol. 145, no. 3, 1 janvier 2008 (2008-01-01), pages 249-257, XP009129049, ISSN: 1423-0097

## Description

L'invention concerne l'utilisation cosmétique et pharmaceutique du microorganisme *Lactobacillus pentosus.*

La peau est un organe en perpétuel renouvellement qui couvre la surface du corps et l'isole de l'environnement extérieur. La peau forme une protection contre les agents externes tels que les assauts chimiques et mécaniques, la température, les infections, l'humidité et les radiations. La peau est structurée en deux compartiments principaux : l'épiderme couvrant la surface de la peau, et le derme profond.

Quoique la structure entière de la peau participe activement dans la défense du corps, le rôle de l'épiderme est essentiel dans la prévention de la perte d'eau et d'autres composants du corps vers son environnement extérieur et dans la protection du corps d'une variété d'agressions environnementales. Sa fonction principale est donc de protéger le corps de menaces extérieures en établissant des barrières physiques, chimiques, biochimiques et immunologiques à leur égard, tout en maintenant une certaine capacité d'échanges entre les milieux extérieur et intérieur.

L'épiderme est un épithélium sous divisé en plusieurs couches ou strates, depuis la couche basale juste au-dessus du derme, en traversant les couches granuleuses et spinales, jusqu'à la couche haute, le *stratum corneum.* Le *stratum corneum* est composé de cellules mortes ne possédant pas de membrane plasmique, ni de noyau, et qui sont encaissées dans une structure appelée enveloppe cornifiée (ou cornée). L'enveloppe cornifiée est hautement résistante et est constituée de protéines structurales et de lipides. Ainsi le *stratum corneum* est la couche de l'épiderme jouant le rôle majeur dans la barrière physique.

En effet le *stratum corneum* constitue une barrière imperméable à l'eau qui empêche la dessiccation. Il doit être correctement hydraté pour assurer sa fonction protectrice, une desquamation normale et rester flexible. Pour cela l'épiderme produit son propre facteur d'hydratation naturelle ou NMF (Natural Moisturizing Factor).

Le NMF est constitué de plusieurs petites molécules, telles que l'urée, des acides aminés, des acides lactiques, des sucres, des ions minéraux. Quelques-unes ont des propriétés hygroscopiques expliquant la capacité à fixer l'eau. D'autres comme le cholestérol fournissent un degré de fluidité et flexibilité à ce qui serait autrement un système de membranes fragile et rigide.

Le NMF représente jusqu'à 20-30 % de la matière sèche du *stratum corneum* et l'aide à rester hydraté. Avec l'âge, la sécheresse de la peau augmente en raison d'une diminution du NMF. De façon similaire, le niveau des composants du NMF est réduit après lavage au savon de la peau. Une réduction du NMF conduit à une peau sèche et à une perturbation de sa fonction barrière. La peau, moins protégée, devient alors beaucoup plus susceptible aux dégâts causés par les agents irritants. Le NMF est donc considéré comme le composant essentiel de la régulation de l'homéostasie épidermique et il existe un besoin de le renforcer.

C'est un objet de la présente invention d'apporter une solution biologique à la protection de la fonction barrière de la peau et à sa réparation suite à une agression extérieure.

Ainsi, l'invention concerne l'utilisation cosmétique ou dans une composition dermatologique à usage topique d'un principe actif provenant d'une culture de *Lactobacillus pentosus.* Dans la présente description, *Lactobacillus pentosus* sera abrégé *L. pentosus.*

*L. pentosus* est une bactérie lactique qui est prévalente dans le milieu de fermentation de l'olive verte dite à l'Espagnole. Cette espèce, probiotique, également présente dans le tube digestif, est connue pour sa capacité à stimuler certains mécanismes immunitaires. A titre d'exemple, elle est rapportée comme favorisant la sécrétion d'immunoglobulines A salivaires chez des patients âgés (Y. Kotani et al. 2011), aussi comme stimulant de l'immunité de type 1, lui conférant un rôle dans la lutte contre certaines infections et allergies (SH Koizumi et al. 2008). Selon le document JP2008013502A, on connait l'utilisation de bactéries lactiques comme L. pentosus en administration par voie orale, pour prévenir ou traiter des infections par Candida.

Les auteurs de la présente invention ont découvert qu'un extrait de culture de *L. pentosus,* en application cutanée, possède des propriétés bénéfiques sur la peau, dont certaines sont complètement inattendues. Celles-ci ont d'abord été observées par la détermination d'un paramètre, la perte d'eau insensible (PEI) qui est un bon indicateur de la fonction barrière de la peau. Il est couramment utilisé pour évaluer les dommages cutanés provoqués par certains agents chimiques, certaines agressions mécaniques ou des conditions pathologiques comme l'eczéma. Ce paramètre a été mesuré dans un test qui sera illustré dans les exemples, et qui révèle une réelle fonction d'amélioration de la fonction barrière d'un extrait de culture, sur une peau présentant une rupture de la barrière causée mécaniquement, à l'aide d'un ruban adhésif.

En poursuivant leurs investigations, les auteurs ont mis en évidence que pour posséder les propriétés attendues, ledit extrait de culture doit réunir et un surnageant, et un lysat cellulaire de la culture.

Un objet de l'invention est donc une utilisation cosmétique d'une association d'un surnageant de culture et d'un lysat cellulaire de *Lactobacillus pentosus,* pour renforcer la fonction barrière du *stratum corneum,* ledit surnageant et ledit lysat cellulaire provenant d'une culture de *Lactobacillus pentosus* en phase stationnaire, après centrifugation du milieu de culture pour obtenir une surnageant et une biomasse, séparation du surnageant et de la biomasse, lyse cellulaire complète de la biomasse pour obtenir le lysat, et mélange dudit surnageant et dudit lysat en un rapport pondéral du surnageant au lysat de 1 à 50. Un autre objet de l'invention est une composition dermatologique à usage topique, comprenant, l'association d'un surnageant de culture et d'un lysat cellulaire, lesdits surnageant et lysat étant issus d'une culture de *L. pentosus,* pour son utilisation dans le traitement de l'état irrité ou inflammatoire de la peau, ledit surnageant et ledit lysat cellulaire provenant d'une culture de *Lactobacillus pentosus* en phase stationnaire, après centrifugation du milieu de culture pour obtenir une surnageant et une biomasse, séparation du surnageant et de la biomasse, lyse cellulaire complète de la biomasse pour obtenir le lysat, et mélange dudit surnageant et dudit lysat dans un rapport pondéral du surnageant au lysat de 1 à 50, ladite association étant présente en une concentration de 0,1 à 10% en poids par rapport au poids total de la composition.

Les auteurs ont découvert que cette association permet de rassembler des protéines, des peptides, des polysaccharides et des acides aminés et organiques à chaînes courtes et plus généralement l'ensemble des composés formant la cellule bactérienne et des métabolites produits par *L. pentosus,* qui, en combinaison, améliorent la fonction barrière du *stratum corneum* ou en accélèrent la récupération lorsqu'elle est altérée, en nourrissant le NMF.

Le principe actif, à savoir l'association précitée, peut être obtenu de différentes manières.

Le surnageant et le lysat cellulaire peuvent provenir de la même culture ; selon cette variante, ils peuvent être directement issus d'un milieu de culture : alors le principe actif est obtenu par mélange de tout ou partie dudit surnageant et tout ou partie du lysat cellulaire; ainsi, il peut se présenter sous la forme d'un extrait total de culture, après que ledit milieu ou ledit extrait ait été soumis à une étape de lyse. Mais de préférence, le rapport pondéral du surnageant au lysat est supérieur à 1. Les conditions de lyse doivent conduire à l'inactivation et la libération des constituants cellulaires des cellules du milieu. La lyse est totale. Ces conditions relèvent des connaissances générales de l'homme du métier, elles entraînent la lyse de toutes les cellules du milieu. Selon une autre variante, le surnageant et le lysat cellulaire peuvent être séparés du milieu de culture, éventuellement traités puis réunis pour obtenir ledit principe actif. Par traitement de l'un et/ou l'autre du surnageant et du lysat cellulaire, on comprend toute opération d'optimisation visant à améliorer leur qualité en vue des propriétés recherchées.

Les milieux de culture adaptés à la croissance de *L. pentosus* comprennent généralement des extraits de levure, des peptones, des sels, des sources inorganiques ou organiques de phosphate, d'azote et de potassium, etc. ainsi que des sucres ; ces milieux, disponibles dans le commerce, ainsi que les conditions de croissance de cette bactérie (pH, température, aération, agitation, potentiel redox, durée) sont des notions bien connues de l'homme du métier. Selon l'invention, un tel milieu peut être mis au point par l'homme du métier, il peut être utilisé tel que disponible dans le commerce ou alors peut être modifié, le plus souvent par la modification de la concentration et/ou de la nature des ingrédients précités, pour favoriser le développement de *L. pentosus.*

Dans la prolongation de leurs travaux, les auteurs ont constaté que ledit principe actif présentait en outre des propriétés bénéfiques sur l'éclat du teint.

L'éclat du teint est d'origine multifactorielle. Il est un mélange pondéré des caractéristiques de la texture de la surface cutanée (par exemple, douce ou rugueuse), de sa brillance, de sa microcirculation et de sa couleur. L'évaluation de l'éclat du teint est généralement faite par l'observation réalisée par des panels d'experts.

Ainsi, dans l'utilisation cosmétique de l'invention, le principe actif précité permet de renforcer l'éclat du teint.

Un principe actif tel que décrit précédemment possède une activité anti-inflammatoire qui résulte de sa capacité à inhiber les enzymes de type lipooxygénases. Les lipooxygénases (LOXs) sont une famille de dioxygénases à fer non héménique, représentant des enzymes clefs dans la biosynthèse de leucotriènes qui sont supposés jouer un rôle important dans la pathophysiologie de plusieurs maladies inflammatoires et allergiques. Les produits issus de l'oxygénation catalysée par les LOXs (acides hydroperoxy/hydroxy eicosatétraénoiques, leucotriènes et lipoxines) sont apparemment impliqués dans le développement de psoriasis et plus généralement dans l'irritation de la peau.

Il ressort de ces propriétés qu'une composition dermatologique de l'invention telle que définie précédemment est destinée à être utilisée dans le traitement, par application topique, des allergies cutanées et des maladies inflammatoires cutanées, comme le psoriasis.

Que ce soit en vue d'une application cosmétique ou dermatologique, par voie cutanée, la concentration du principe actif varie avantageusement de 0,1 à 10% en poids par rapport au poids total de la composition.

La production du principe actif comprend les étapes suivantes :
Pour une production optimale de *L. pentosus,* on cultive avantageusement la bactérie, jusqu'à un stade avancé de la phase exponentielle de la croissance microbienne, de préférence en phase stationnaire. Un milieu particulièrement adapté à l'obtention d'un principe actif efficace est choisi parmi les milieux M20 et MRS, commercialisés ainsi que ces mêmes milieux dont la concentration et/ou la nature des ingrédients peuvent être modifiés pour favoriser le développement de *L. pentosus.* Le culot et le surnageant sont alors récupérés. L'étape de récupération est classiquement réalisée et les techniques de routine en microbiologie sont appropriées. Ainsi, on peut séparer le surnageant du milieu de culture par filtration ou centrifugation. La biomasse microbienne résultante est traitée afin d'obtenir un lysat de cellules.

Un lysat désigne communément un matériau obtenu à l'issue de la destruction ou dissolution de cellules biologiques par un phénomène dit de lyse cellulaire provoquant ainsi la libération des constituants biologiques intracellulaires naturellement contenus dans les cellules du microorganisme considéré. Au sens de la présente invention, le terme lysat est utilisé indifféremment pour désigner l'intégralité du lysat tel que défini ci-dessus ou une fraction de celui-ci, ce lysat étant brut ou ayant subi un ou plusieurs traitements, ceux-ci ne devant pas affecter sensiblement les propriétés du lysat dans un principe actif utilisé selon l'invention. Le lysat mis en oeuvre est donc formé en tout ou partie des constituants biologiques intracellulaires et des constituants des parois et membranes cellulaires. Avantageusement, un lysat utilisé pour l'invention est constitué de l'intégralité du lysat obtenu. La lyse cellulaire peut être accomplie par différentes technologies, telles qu'un choc osmotique, un choc thermique, les ultrasons ou encore une contrainte mécanique de type centrifugation.

Le lysat et le surnageant de culture sont alors mélangés. Le rapport pondéral du surnageant au lysat varie de 1 à 50, de préférence de 5 à 15. Le principe actif obtenu peut être mis en oeuvre sous différentes formes telles qu'une solution, une poudre éventuellement lyophilisée atomisée ou concentrée.

Dans le cadre de la présente invention, le principe actif peut être formulé sous toute forme galénique appropriée à son administration, par exemple sous forme de crème, gel, lotion, lait, émulsion huile dans eau ou eau dans huile, solution, onguent, pulvérisateur, huile corporelle, lotion après-rasage, savon, bâton protecteur des lèvres, bâton et crayon pour maquillage, aérosol, roll-on, stick, bille, poudre, lingette, incorporation dans des vecteurs de type liposomes, glycosphères, cyclodextrines, dans des chylomicrons, des macro-, micro-, nano-particules ainsi que les macro-, micro- et nanocapsules et aussi adsorbtion sur des polymères organiques poudreux, des talc, bentonites et autres supports minéraux.

Ledit principe actif peut être utilisé avec des additifs ou des adjuvants usuels en cosmétologie, comme par exemple des agents antimicrobiens ou des parfums mais aussi des lipides d'extraction ou de synthèse, des polymères gélifiants et viscosifiants, des tensio-actifs et des émulsifiants, des principes actifs hydro- ou liposolubles, des extraits de plantes, des extraits tissulaires, des extraits marins, des actifs de synthèse.

Il peut aussi être utilisé avec d'autres principes actifs complémentaires choisis pour leur action, par exemple pour l'effet amincissant, l'effet anti-cellulite, l'effet raffermissant, l'effet hydratant, l'effet anti-âge, l'effet éclaircissant, l'effet sur la coloration de la peau, l'activité antimicrobienne, l'activité anti-oxydante, l'activité anti-radicalaire, l'effet cicatrisant, l'effet tenseur, l'effet anti-ride, l'activité chélatante, l'activité complexante et séquestrante, l'effet apaisant, l'effet anti-cernes, l'effet anti-rougeurs, l'activité émolliente, l'effet démêlant capillaire, l'activité anti-pelliculaire, l'effet stimulant de la repousse du cheveu, l'effet inhibant la chute du cheveu, l'effet gainant capillaire, l'activité épilatoire, l'activité limitant la repousse du poil, l'activité participant au renouvellement cellulaire, l'activité modulant la réponse inflammatoire, l'activité participant au maintien de l'ovale du visage, mais également la protection solaire, l'activité anti-irritante, la nutrition cellulaire, la respiration cellulaire, les traitements anti-séborrhéiques, la tonicité cutanée, la protection du cheveu.

Lorsque ledit principe actif est utilisé avec des principes actifs complémentaires, ceux-ci sont généralement présents à une concentration suffisamment élevée pour qu'ils puissent exercer leur activité.

Selon la présente invention, l'utilisation est de préférence quotidienne et l'application de une ou plusieurs fois par jour. Elle est très bien tolérée, elle ne présente aucune toxicité et, pour des périodes de temps prolongées, n'implique aucun effet systémique.

Il est ci-après décrit un procédé avantageux de préparation d'un principe actif cosmétique ou dermatologique à usage topique, à base d'un milieu de culture de *L. pentosus.* Ce procédé qui n'est pas un objet de l'invention comprend les étapes suivantes :
On obtient une culture de *L. pentosus* jusqu'en phase stationnaire,
On centrifuge le milieu de culture afin d'obtenir un surnageant de culture et une biomasse microbienne,
On sépare le surnageant de la biomasse,
On réalise une lyse cellulaire de la biomasse pour obtenir un lysat cellulaire, et
On mélange le surnageant et le lysat.
Le rapport pondéral du surnageant au lysat varie de 1 à 50.

Bien entendu, toute étape complémentaire, par exemple de traitement de l'un et/ou l'autre du surnageant et de la biomasse, bien connu de l'homme du métier, peut être effectuée, pour obtenir un principe actif tel que décrit précédemment.

L'invention concerne aussi la souche *L. pentosus* CNCM I-4730 telle que déposée le 4 avril 2013 conformément au traité de Budapest auprès de la Collection nationale de Culture de Microorganismes (CNCM).

La présente invention est maintenant illustrée, de manière non limitative, par les exemples suivants et la figure jointe.

La Figure illustre l'activité anti-inflammatoire d'un extrait de culture utilisé selon la présente invention, par l'analyse de la libération et/ou synthèse de l'interleukine 8 (en pg/mL) par des épidermes reconstruits stimulés.

### Exemple 1 : Culture de L. pentosus CNCM I-4730

La souche *L. pentosus* CNCM I-4730 est produite en fermenteur de 80 L dans le milieu de culture présenté tableau 1. Le milieu est ensemencé avec un inoculum de 3 % (volume/volume) à partir d'une culture de *L. pentosus* CNCM I-4730 âgée de 24 h. La croissance de la souche de *L. pentosus* CNCM I-4730 est réalisée à 30°C avec une agitation de 50 tours/min sans arrivée d'air, ni régulation pH. La culture est conduite jusqu'en phase stationnaire soit 20 heures après son ensemencement.

**Tableau 1 : milieu de culture de L. pentosus CNCM I-4730**

| Milieu de culture | |
|---|---|
| Ingrédient | Concentration en g/l de milieu de culture |
| Extrait de levure | 15,00 |
| Glucose | 20,00 |
| Tween® 80 | 1,08 |
| K₂HPO₄ | 2,00 |
| Acétate de sodium | 5,00 |
| Citrate d'ammonium | 2,00 |
| MgSO₄ | 0,20 |
| MnSO₄ | 0,05 |

### Exemple 2: Préparation du principe actif utilisé selon l'invention

La culture obtenue dans les conditions décrites à l'Exemple 1 est totalement centrifugée sur centrifugeuse en continu, du type Sharpless, à 15.000 tours/min afin de séparer la biomasse microbienne et le surnageant de culture. Puis la biomasse cellulaire et le surnageant sont traités comme suit.

### Préparation du lysat de cellules de L. pentosus CNCM I-4730

La biomasse microbienne est récupérée (environ 900 g de culot à 21-25% de matière sèche) puis remise en suspension dans 5 volumes d'eau et centrifugée à 4.000 tours/minute durant 30 minutes. Après élimination de l'eau, la biomasse est récupérée. La biomasse ainsi lavée, est diluée dans une solution 2 M H₂SO₄ avec un ratio massique 50/50. La préparation est chauffée à 100°C durant 1h30 min afin de réaliser la lyse des cellules bactériennes.

### Préparation du surnageant

60 L de surnageant issus de la centrifugation Sharpless ci-dessus sont filtrés à 0,2 µm sur un filtre plaque. Le filtrat est récupéré.

### Préparation de l'extrait de L. pentosus CNCM I-4730

Afin d'obtenir l'extrait de culture selon la présente invention, un volume du lysat de cellules de *L. pentosus* CNCM I-4730 est mélangé avec 9 volumes de surnageant, puis le pH est ajusté à 4 avec du NaOH.

### Exemple 3 : Formulation possible de l'actif à base de l'extrait de Lactobacillus pentosus CNCM I-4730 obtenue selon l'exemple 2

Le principe actif obtenu à l'Exemple 2 est formulé à pH 5,5 selon la composition suivante, en pourcentage massique :

| | |
|---|---|
| Eau | 89.39 |
| SEPIGEL™ 305 | 2.00 |
| LABRAFAC™ CC | 5.00 |
| MICROCARE® PM4 | 1.00 |
| SILK&CARE | 0.50 |
| Extrait L. pentosus | 2.00 |
| NaOH 10% | 0.09 |
| Acide citrique 10% | 0.02 |
| Total | 100.00 |

### Exemple 4 : Activité anti-inflammatoire

### Activité anti-inflammatoire évaluée in vitro par un test d'inhibition de l'activité de la 5-lipoxygénase (LOX-5)

La LOX-5 est une enzyme impliquée dans le processus pro-inflammatoire en permettant la formation de leucotriènes inflammatoires à partir de l'acide arachidonique.

L'activité anti-inflammatoire d'un extrait de culture, obtenu à l'Exemple 2, est évaluée, *in vitro,* par sa capacité à inhiber la LOX-5. Celle-ci est estimée, par spectrophotométrie (à 233 nm), par l'inhibition de la transformation de l'acide linoléique en acide hydroxyperoxylinoléique. Le suivi de l'inhibition de l'activité LOX-5 par différentes concentrations de l'extrait de culture permet de déterminer l'IC₅₀ de cet extrait, c'est-à-dire la concentration dudit extrait de culture nécessaire pour induire une inhibition de 50% de l'activité LOX-5.

Pour cela, dans un milieu réactionnel (3 mL) contenant 2950 µL de tampon phosphate (0,1 M, pH=7,4), 1 000 unités de l'enzyme LOX-5 (10 µL) et 50 µM d'acide linoléique (10 µL), sont ajoutés 30 µL de l'extrait de culture obtenu ci-dessus à différentes dilutions (0 - 16,8 - 56 - 112 mg matière sèche/mL d'extrait).

Les résultats de cette étude ont permis de mettre en évidence une activité anti-inflammatoire de l'extrait de culture, par inhibition de l'activité 5-lipoxygénase avec une IC₅₀ de 0,674 mg de matière sèche/mL d'extrait.

### Activité anti-inflammatoire évaluée in vitro sur la synthèse et la libération de l'interleukine 8 (IL-8) par des épidermes reconstruits stimulés

Des épidermes reconstruits ont été stimulés par le phorbol 12-myristate 13-acetate (PMA), un agent pro-inflammatoire. La stimulation des épidermes reconstruits par le PMA à 0,3 µg/mL pendant 24h provoque un état inflammatoire et génère une importante libération et/ou synthèse d'IL-8.

La libération et/ou synthèse d'IL-8 ont été évaluées par la méthode immuno-enzymatique ELISA, à partir des surnageants de culture des épidermes reconstruits comme suit :
- épidermes non traités,
- épidermes traités avec le PMA pour mimer l'état inflammatoire,
- épidermes pré-traités avec la dexaméthasone (10⁻⁷M), une hormone glucocorticoïde de synthèse servant de contrôle), puis traités avec le PMA,
- épidermes pré-traités avec l'extrait de culture obtenu ci-dessus, à différentes concentrations (0,112 et 0,280 mg matière sèche/mL d'extrait), puis traités avec le PMA.

La pré-incubation des épidermes reconstruits avec la dexaméthasone a inhibé la libération et/ou synthèse d'IL-8 de 2,4 fois.

La pré-incubation des épidermes reconstruits avec l'extrait de culture obtenu à l'Exemple 2, à 0,112 mg/mL et 0,280 mg/mL, a significativement diminué de 1,8 fois et 2,4 fois la libération et/ou synthèse d'IL-8, respectivement.

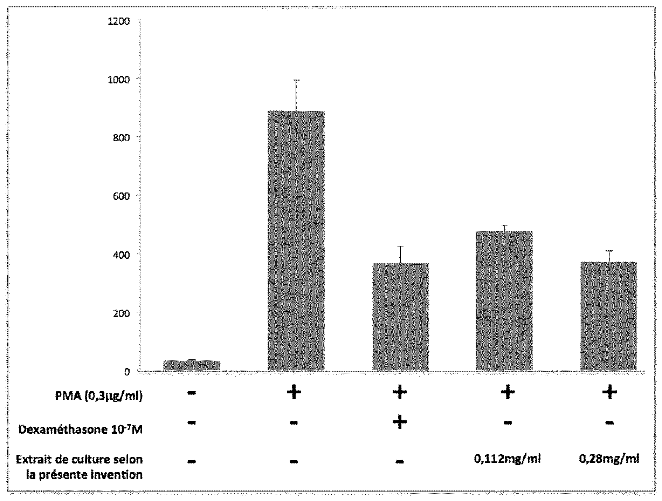

Les résultats de cet essai sont présentés à la Figure 1. Ils ont permis de mettre en évidence une puissante activité anti-inflammatoire de l'extrait de culture obtenu pour la présente invention. En effet, l'extrait de culture aux concentrations de 0,112 mg et 0,280 mg de matière sèche/mL d'extrait, a inhibé la libération et/ou la synthèse d'IL-8 induite par l'agent pro-inflammatoire PMA. On observe que cette activité approche (pour la concentration de 0,112 mg/mL) et est similaire (pour la concentration de 0,280 mg/mL) à celle de la dexaméthasone.

### Exemple 5 : Effet de protection de la fonction barrière cutanée

Une étude clinique a été réalisée en double aveugle sur 8 volontaires humains (8 femmes de type caucasien âgées de 18 à 69 ans) afin d'estimer, au niveau de l'avant-bras, l'effet protecteur de la fonction barrière cutanée d'un principe actif selon la présente invention préparé selon l'Exemple 2.

Chaque volontaire a appliqué, sur l'un de ses avant-bras, une formulation cosmétique contenant 2%, en poids, dudit principe actif (soit 2 g d'extrait de culture pour 100 g de composition), et sur son autre avant-bras, une formulation cosmétique placébo (formulation de composition identique mais ne contenant pas d'extrait de culture pour la présente invention). La répartition des avant-bras a été réalisée de façon randomisée. Quinze minutes après l'application de ces formulations, la fonction barrière cutanée des deux avant-bras est altérée par une méthode dite de « *tape-stripping* » définie par 8 cycles successifs, de 2 secondes chacun, d'application / retrait d'une bande adhésive.

L'évaluation de la fonction barrière cutanée est réalisée avant l'application des formulations et 30 minutes après l'agression cutanée par *« tape-stripping* », par la mesure de la perte en eau transépidermique (*TEWL* = *TransEpidermal Water Loss*) grâce à l'utilisation d'un Tewameter™ TM 300 (Commercialisé par Courage + Khazaka electronic). La mesure de la TEWL permet d'estimer le degré d'évaporation d'eau diffusant à travers le *stratum corneum* (g.m⁻².h⁻¹). Une altération de la fonction barrière cutanée induit une perte transépidermique d'eau donc une augmentation de la TEWL. Plus la TEWL est faible et plus la fonction barrière est préservée.

Les résultats de cette étude sont présentés dans le tableau 2 ci-dessous.

**Tableau 2**

| ***Pourcentage de variation de la TEWL 30 minutes après l'agression cutanée*** | |
|---|---|
| Formulation Placébo | Formulation avec l'extrait de culture à 2 % |
| 13,8 ± 4 | 4 ± 0,7 |

L'extrait de culture pour la présente invention utilisé à 2% en formulation cosmétique présente une activité de protection de la fonction de la barrière cutanée. En effet, la perte en eau transépidermique (TEWL) mesurée 30 minutes après l'agression cutanée n'est augmentée que de 4 ± 0,7 % chez les personnes ayant reçu une application de la formulation contenant l'extrait de culture pour la présente invention, alors que cette augmentation est de 13,8 ± 4 % chez les personnes ayant reçu une application de la formulation placébo.

L'extrait de culture présente un effet protecteur sur la fonction barrière cutanée.

### Exemple 6 : Effet de réparation de la fonction barrière cutanée

Une étude clinique a été réalisée en double aveugle sur 14 volontaires humains (14 femmes de type caucasien âgées de 18 à 69 ans) afin d'estimer, au niveau de l'avant-bras, l'effet réparateur de la fonction barrière cutanée (après une agression cutanée par « *tape-stripping* ») d'un principe actif pour la présente invention. Durant 13 jours (J-7 à J+6), deux fois par jour, chaque volontaire a appliqué, sur l'un de ses avant-bras une formulation cosmétique contenant 2%, en poids, d'extrait de culture pour la présente invention (soit 2 g d'extrait de culture pour 100 g de formulation), tel que préparé à l'Exemple 2 ; sur son autre avant-bras une formulation cosmétique placébo (formulation de composition identique mais ne contenant pas d'extrait de culture). La répartition des avant-bras a été réalisée de façon randomisée. A J0, après 7 jours d'application des deux types de formulations, la fonction barrière cutanée des deux avant-bras est altérée par « *tape-stripping* » (8 cycles successifs, de 2 secondes chacun, d'application / retrait d'une bande adhésive). Le suivi de la fonction barrière cutanée est réalisé à J0 après l'agression cutanée, à J+1, à J+2 et à J+6, par la mesure de la perte en eau transépidermique (*TEWL* = *TransEpidermal Water Loss*) grâce à l'utilisation d'un Tewameter™ TM 300 (Commercialisé par Courage + Khazaka electronic). La mesure de la TEWL permet d'estimer le degré d'évaporation d'eau diffusant à travers le *stratum corneum* (g.m⁻².h⁻¹). Une altération de la fonction barrière cutanée induit une perte transépidermique d'eau donc une augmentation de la TEWL. Plus la TEWL est faible et plus la fonction barrière est préservée. L'effet de réparation de la fonction barrière cutanée est déterminé par le suivi du pourcentage de variation de la TEWL au cours du temps après l'agression cutanée, en prenant comme référence la mesure de la TEWL réalisée juste après l'agression cutanée. L'effet réparateur correspondra à une diminution dans le temps de ce pourcentage de variation de la TEWL.

Les résultats de cette étude sont présentés dans le tableau 3 ci-dessous.

**Tableau 3**

| ***Pourcentage de variation de la TEWL au cours du temps après l'agression cutanée*** | | | |
|---|---|---|---|
| | à J+1 | à J+2 | à J+6 |
| Formulation Placébo | 12,9 ± 3 | 8,5 ± 1,3 | -1,8 ± 0,3 |
| Formulation avec 2% de l'extrait de culture selon la présente invention | 5,9 ± 1,1 | -1,1 ± 0,3 | -5,4 ± 1,1 |

Ces résultats confirment qu'un principe actif utilisé selon la présente invention présente un effet protecteur sur la fonction barrière cutanée car un jour (J+1) après l'agression cutanée, le pourcentage de variation de la TEWL n'augmente que de 5,9 ± 1,1 % chez les personnes ayant reçu la formulation contenant l'extrait de culture pour la présente invention, en comparaison à l'augmentation de 12,9 ± 3 % chez les personnes ayant reçu la formulation placébo.

L'extrait de culture pour la présente invention accélère et amplifie la récupération de la fonction barrière cutanée. En effet, dès deux jours (J+2) après l'agression cutanée, le pourcentage de variation de la TEWL est négatif (-1,1 ± 0,3 %) signifiant que la perte en eau transépidermique (TEWL) est inférieure à celle mesurée juste après l'agression cutanée (valeur de référence). Six jours (J+6) après l'agression cutanée, la récupération de la fonction barrière cutanée est 2,8 fois plus importante chez les personnes ayant reçu la formulation contenant l'extrait de culture utilisé selon la présente invention (pourcentage de variation de la TEWL de -5,4 ± 1,1 %) par rapport aux personnes ayant reçu la formulation placébo (pourcentage de variation de la TEWL de -1,8 ± 0,3 %).

L'extrait de culture utilisé selon la présente invention accélère et amplifie la réparation de la fonction barrière cutanée.

### BIBLIOGRAPHIE

Y. Kotani et al. : Oral intake of Lactobacillus pentosus strain b240 accelerates salivary immunglobulin A secretion in the eldery : A randomized, placebo-controlled, double-blind trial, Immunity & Ageing 2010, 7:11, 1-11.
SH Koizumi et al. : Essential role of Toll-like receptors for dendritic cell and NK1.1+ cell-dependent activation of type 1 immunity by Lactobacillus pentosus strain S-PT84, Immunology Letters 2008, 120, 14-19.

## Revendications

1. Utilisation cosmétique d'une association d'un surnageant de culture et d'un lysat cellulaire de *Lactobacillus pentosus,* pour renforcer la fonction barrière du *stratum corneum,* ledit surnageant et ledit lysat cellulaire provenant d'une culture de *Lactobacillus pentosus* en phase stationnaire, après centrifugation du milieu de culture pour obtenir une surnageant et une biomasse, séparation du surnageant et de la biomasse, lyse cellulaire complète de la biomasse pour obtenir le lysat, et mélange dudit surnageant et dudit lysat en un rapport pondéral du surnageant au lysat de 1 à 50.

2. Utilisation selon la revendication 1, **caractérisé en ce que** le rapport pondéral du surnageant au lysat varie de 5 à 15.

3. Utilisation selon la revendication 1 ou 2, pour renforcer l'éclat du teint de la peau.

4. Utilisation selon l'une quelconque des revendications 1 à 3, **caractérisée en ce que** le surnageant et le lysat proviennent de la même culture.

5. Utilisation selon l'une quelconque des revendications 1 à 4, **caractérisée en ce que** la ou les cultures de *Lactobacillus pentosus* est/sont celle(s) de la souche *Lactobacillus pentosus* CNCM I-4730 telle que déposée le 4 avril 2013 conformément au traité de Budapest auprès de la Collection nationale de Culture de Microorganismes (CNCM)

6. Composition dermatologique à usage topique comprenant l'association d'un surnageant de culture et d'un lysat cellulaire, issus d'une culture de *Lactobacillus pentosus,* pour son utilisation dans le traitement de l'état irrité ou inflammatoire de la peau, ledit surnageant et ledit lysat cellulaire provenant d'une culture de *Lactobacillus pentosus* en phase stationnaire, après centrifugation du milieu de culture pour obtenir une surnageant et une biomasse, séparation du surnageant et de la biomasse, lyse cellulaire complète de la biomasse pour obtenir le lysat, et mélange dudit surnageant et dudit lysat dans un rapport pondéral du surnageant au lysat de 1 à 50, ladite association étant présente en une concentration de 0,1 à 10% en poids par rapport au poids total de la composition.

7. Composition selon la revendication 6, **caractérisée en ce que** le rapport pondéral du surnageant au lysat varie de 5 à 15.

8. Souche *Lactobacillus pentosus* CNCM I-4730 telle que déposée le 4 avril 2013 conformément au traité de Budapest auprès de la Collection nationale de Culture de Microorganismes (CNCM).

## Patentansprüche

1. Kosmetische Verwendung einer Verbindung eines Kulturüberstands und eines Zelllysats von *Lactobacillus pentosus,* um die Barrierefunktion des *stratum corneum* zu stärken, wobei der Überstand und das Zelllysat aus einer Kultur von *Lactobacillus pentosus* in stationärer Phase stammen, nach der Zentrifugation des Kulturmediums, um einen Überstand und eine Biomasse zu erhalten, Trennen des Überstands und der Biomasse, vollständige Zelllyse der Biomasse, um das Lysat zu erhalten, und Mischen des Überstands und des Lysats in einem Gewichtsverhältnis des Überstands zum Lysat von 1 bis 50.

2. Verwendung nach Anspruch 1, **dadurch gekennzeichnet, dass** das Gewichtsverhältnis des Überstands zum Lysat von 5 bis 15 variiert.

3. Verwendung nach Anspruch 1 oder 2, um den Glanz des Teints der Haut zu verstärken.

4. Verwendung nach einem der Ansprüche 1 bis 3, **dadurch gekennzeichnet, dass** der Überstand und das Lysat aus der gleichen Kultur stammen.

5. Verwendung nach einem der Ansprüche 1 bis 4, **dadurch gekennzeichnet, dass** die Kultur(en) von *Lactobacillus pentosus* diejenige(n) des Stamms *Lactobacillus pentosus* CNCM I-4730 ist/sind, wie am 4. April 2013 gemäß dem Vertrag von Budapest in der Collection nationale de Culture de Microorganismes (CNCM) hinterlegt.

6. Dermatologische Zusammensetzung zur topischen Verwendung, umfassend die Verbindung eines Kulturüberstands und eines Zelllysats, die aus einer Kultur von *Lactobacillus pentosus* stammen, für ihre Verwendung bei der Behandlung des gereizten oder entzündeten Zustands der Haut, wobei der Überstand und das Zelllysat aus einer Kultur von *Lactobacillus pentosus* in stationärer Phase stammen, nach der Zentrifugation des Kulturmediums, um einen Überstand und eine Biomasse zu erhalten, Trennen des Überstands und der Biomasse, vollständige Zelllyse der Biomasse, um das Lysat zu erhalten, und Mischen des Überstands und des Lysats in einem Gewichtsverhältnis des Überstands zum Lysat von 1 bis 50, wobei die Verbindung in einer Konzentration von 0,1 bis 10 Gew% mit Bezug auf das Gesamtgewicht der Zusammensetzung vorhanden ist.

7. Zusammensetzung nach Anspruch 6, **dadurch gekennzeichnet, dass** das Gewichtsverhältnis des Überstands zum Lysat von 5 bis 15 variiert.

8. Stamm *Lactobacillus pentosus* CNCM I-4730, wie am 4. April 2013 gemäß dem Vertrag von Budapest in der Collection nationale de Culture de Microorganismes (CNCM) hinterlegt.

## Claims

1. A cosmetic use of an association of a culture supernatant and a cell lysate of *Lactobacillus pentosus,* to reinforce the barrier function of the *stratum corneum,* said supernatant and said cell lysate originating from a *Lactobacillus pentosus* culture in the stationary phase, after a centrifugation of the culture medium to obtain a supernatant and a biomass, a separation of the supernatant and the biomass, a complete cell lysis of the biomass to obtain the lysate, and a mixture of said supernatant and said lysate in a weight ratio of the supernatant to the lysate of 1 to 50.

2. The use according to claim 1, **characterized in that** the weight ratio of the supernatant to the lysate ranges from 5 to 15.

3. The use according to claim 1 or 2, to enhance the radiance of the complexion of the skin.

4. The use according to any one of claims 1 to 3, **characterized in that** the supernatant and the lysate originate from the same culture.

5. The use according to any one of claims 1 to 4, **characterized in that** the *Lactobacillus pentosus* culture(s) is/are that/those of the *Lactobacillus pentosus* strain CNCM 1-4730 as filed on April 4^{th}, 2013 in accordance with the Budapest treaty before the National Collection of Microorganism Culture (CNCM).

6. A dermatological composition for topical use comprising the association of a culture supernatant and a cell lysate, derived from a *Lactobacillus pentosus* culture, for the use thereof in the treatment of the irritated or inflammatory state of the skin, said supernatant and said cell lysate originating from a *Lactobacillus pentosus* culture in the stationary phase, after a centrifugation of the culture medium to obtain a supernatant and a biomass, a separation of the supernatant and the biomass, a complete cell lysis of the biomass to obtain the lysate, and a mixture of said supernatant and said lysate in a weight ratio of the supernatant to the lysate of 1 to 50, said association being present in a concentration of 0.1 to 10% by weight relative to the total weight of the composition.

7. The composition according to Claim 6, **characterized in that** the weight ratio of the supernatant to the lysate ranges from 5 to 15.

8. The *Lactobacillus pentosus* strain CNCM 1-4730 as filed on April 4^{th}, 2013 in accordance with the Budapest treaty before the National Collection of Microorganism Culture (CNCM).
